# FASCICULE DE BREVET EUROPEEN

(11) **EP 0 712 855 B1**
(45) Date de publication et mention de la délivrance du brevet: **17.09.1997**
(21) Numéro de dépôt: 95402307.3
(22) Date de dépôt: 16.10.1995
(51) Int. Cl.: C07F 7/08, A61K 7/42

(54) **Filtres solaires, compositions cosmétiques photoprotectrices les contenant et utilisations**
Sonnenschutzfilter, kosmetische Sonnenchutzmittel, die sie enthalten, und Verwendungen
Solar filters, cosmetic photoprotective compositions and their use

(30) Priorité: 17.11.1994 FR 9413771
(43) Date de publication de la demande: 22.05.1996
(73) Titulaire: L'OREAL, 75008 Paris (FR)
(72) Inventeur: Richard, Hervé, F-93420 Villepinte (FR); Leduc, Madeleine, F-75011 Paris (FR); Lagrange, Alain, F-77700 Couvray (FR)
(74) Mandataire: Andral, Christophe André Louis

(56) Documents cités:
- EP-A- 0 335 777
- EP-A- 0 389 337
- EP-A- 0 392 883
- WO-A-92/19625
- GB-A- 2 157 174

## Description

La présente invention concerne de nouveaux composés du type diorganosiloxanes, à chaines courtes, linéaires ou cycliques, ou du type triorganosilanes, présentant pour caractéristique commune d'avoir tous au moins un motif filtrant particulier et convenablement sélectionné à fonction sulfonamide, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinées à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (lP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de l'acide p- aminobenzoïque, les dérivés du benzylidène camphre et en particulier les dérivés du 3-benzylidène camphre, les dérivés de l'acide cinnamique et les dérivés du benzotriazole. Cependant, certaines de ces substances ne présentent pas toutes les propriétés requises pour une utilisation convenable comme filtres UV dans les compositions antisolaires. En particulier, leur pouvoir filtrant intrinsèque peut être insuffisant, leur solubilité dans les différents type de formulations utilisés en matière de protection solaire n'est pas toujours suffisamment bonne (liposolubilité notamment), elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

Ainsi, dans le cas plus particulier des substances filtres de type benzotriazole ou 3-benzylidène camphre, on a cherché à obtenir des produits à propriétés améliorées, en particulier au niveau de leur liposolubilité et de leur caractère cosmétique, en venant fixer par greffage (hydrosilylation) le groupement filtrant benzotriazole ou 3-benzylidène camphre sur une chaine macromoléculaire de type silicone (organopolysiloxane) au moyen d'un chainon de raccordement de type alkylène ou alkylèneoxy. Cette technique, décrite respectivement dans les demandes de brevets EP-A- 0 392 883 (benzotriazole) et EP-A- 0 335 777 (3-benzylidène camphre) toutes deux au nom de la Demanderesse, conduit certes à des composés intéressants (ces produits sont connus sous le nom général de "silicones filtres"), mais le caractère liposoluble de ces derniers peut encore apparaitre comme insuffisant et, de plus, afin d'obtenir des propriétés filtrantes satisfaisantes avec ce type de produits, il est souvent nécessaire de mettre en oeuvre des quantités relativement importantes de ces polymères filtres, ce qui se traduit par de mauvaises propriétés cosmétiques au niveau des formulations qui les contiennent.

La présente invention vise à résoudre les problèmes ci-dessus en proposant de nouveaux composés, de type silicones filtres, à motifs filtrants particuliers, qui présentent des propriétés améliorées, notamment au niveau de leur solubilité dans les corps gras et de leurs propriétés cosmétiques.

Plus précisemment encore, il a été trouvé, selon la présente invention, qu'en venant fixer par greffage, notamment par réaction d'hydrosylilation, un ou plusieurs motifs filtrants particuliers, à savoir des motifs filtrants choisis parmi les dérivés de 3-benzylidène camphre, les benzotriazoles, les benzophénones et les benzimidazoles, sur une chaine silicone convenablement sélectionnée, linéaire ou cyclique, ou sur un silane convenablement sélectionné, et ceci par l'intermédiaire d'un chainon de raccordement spécifique à fonction sulfonamide, il était possible d'aboutir à de nouveaux composés du type silicones filtres obviant aux inconvénients des silicones filtres de l'art antérieur, ces nouveaux composés présentant en particulier de très bonnes propriétés filtrantes, soit dans l'UV-A soit dans l'UV-B selon leur structure chimique, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés qui sont caractérisés par le fait qu'ils répondent à l'une des formules (1) à (3) suivantes : 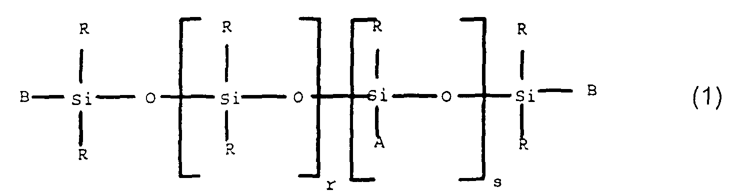 ou 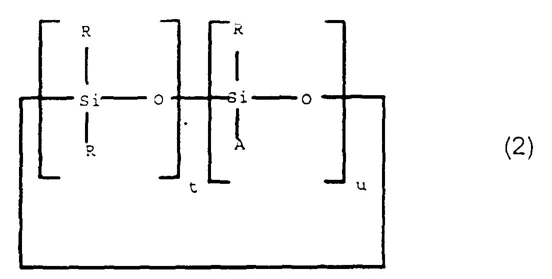 ou

A-Si(R')₃ (3)

formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical lié directement à un atome de silicium, ledit radical A répondant à l'une des formules (4.1) à (4.4) suivantes : 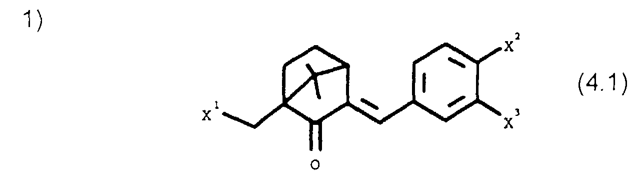 formule (4.1) dans laquelle :
   ∗ **X**^{**1**} représente un atome d'hydrogène, ou un radical divalent -Y- de formule (5) suivante : 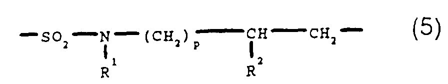 formule (5) dans laquelle :
      - R¹ désigne un atome d'hydrogène, un radical alkyle ou hydroxyalkyle en C₁-C₄,
      - R² représente un atome d'hydrogène, un radical alkyle en C₁-C₆ ou alcoxy en C₁-C₆,
      - p est un nombre entier compris entre 1 et 10 inclusivement,
      - le groupement -CH₂- terminal est directement lié à un atome de silicium ;
   ∗ **X**^{**2**} et **X**^{**3**}, identiques ou différents, représentent un atome d'hydrogène ou d'halogène, un radical alkyle ou alcoxy en C₁-C₄, un radical divalent -Y-, ou un radical Z de formule (6) suivante : 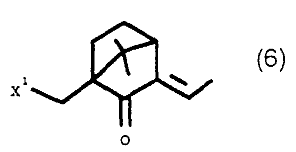 ou encore peuvent former ensemble un groupement alkylidène dioxy dans lequel le groupe alkylidène contient 1 ou 2 atomes de carbone;
   étant entendu que dans cette formule (4.1) :
   - l'un des trois symboles X¹, X² et X³ doit nécessairement représenter un radical divalent -Y-, et aucun des deux autres symboles restants ne peut alors représenter un radical divalent -Y-,
   - si X¹ représente un atome d'hydrogène, alors X² et X³ sont nécessairement différents et aucun ne peut représenter un radical Z,
   - si X¹ représente un radical divalent -Y-, alors X² et X³ ne peuvent pas simultanément représenter un radical Z,
   - si X¹ désigne un atome d'hydrogène et X³ représente un radical divalent -Y-, alors X² est de préférence différent d'un atome d'hydrogène ; 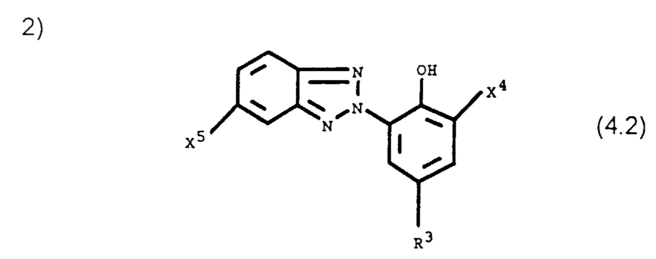 formule (4.2) dans laquelle :
      ∗ **X**^{**4**} représente un atome d'hydrogène, un radical alkyle en C₁-C₈ ou un radical divalent-Y-,
      ∗ **X**^{**5**} représente un atome d'hydrogène ou d'halogène, un radical alkyle ou alcoxy en C₁-C₄ ou un radical divalent -Y-,
      ∗ **R**^{**3**} représente un radical alkyle en C₁-C₈,
   étant entendu que dans cette formule (4.2) l'un des deux symboles X⁴, X⁵ doit nécessairement représenter un radical divalent -Y-, le symbole restant ne pouvant pas représenter un radical divalent -Y- ;

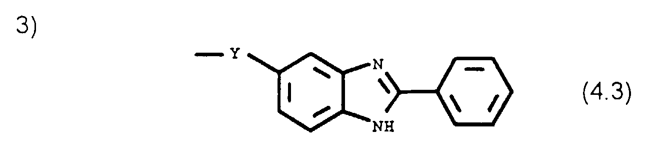

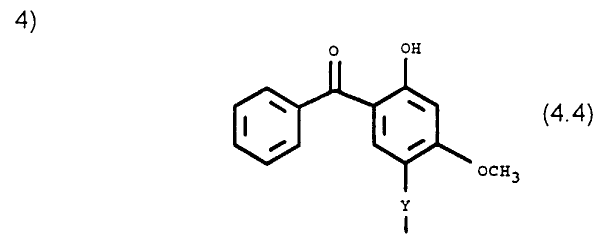

Dans les formules (1) à (3) ci-dessus, A représente donc un groupement filtrant, sélectionné au sein des dérivés du 3-benzylidène camphre (formule 4.1), des benzotriazoles (formule 4.2), des benzimidazoles (formule 4.3) et des benzophénones (formule 4.4) qui, après fixation sur la chaine courte siliconée de départ ou sur le silane de départ, confère aux composés de type diorganosiloxanes linéaires (formule (1)) ou cycliques (formule (2)) ou de type triorganosilanes (formule (3)) des propriétés absorbantes vis à vis de l'ultraviolet dans une zone de longueur d'onde pouvant aller de 280 à 400 nm. Comme indiqué précédemment, et comme cela ressort des définitions des formules (4.1) à (4.4) donnée ci-dessus, ce groupement présente nécessairement au moins une fonction sulfonamide (formule 5) qui est apportée par le chainon qui assure l'accrochage du motif filtrant à la chaine silicone ou au silane. L'un des avantages qui est attaché aux composés selon l'invention, et plus particulièrement à ceux pour lesquels le motif filtrant A répond à la formule (4.1) ou à la formule (4.2), est que l'on peut obtenir, selon la nature et/ou la position des différents subsituants portés sur ce motif filtrant A, des filtres soit purement UV-A ou au contraire purement UV-B, et ceci avec des coefficients d'extinction particulièrement élevés.

Comme cela ressort des définitions données ci-avant, l'accrochage du chainon -SO₂-N(R¹)-(CH₂)ₚ-CH(R²)-CH₂- (c'est à dire du radical divalent -Y- de formule (5) possédant la fonction sulfonamide) sur un motif filtrant dérivé du 3-benzylidène camphre, qui assure donc le raccordement dudit motif à l'atome de silicium de la chaine siliconée ou du silane, peut, selon la présente invention, se faire à l'une quelconque des positions occupées par les symboles X¹, X² et X³, la partie terminale -SO₂- dudit chainon venant se fixer sur le motif dérivé du 3-benzylidène camphre et sa partie terminale -CH₂- sur un atome de silicium de la chaine siliconée ou du silane. Toujours dans le cas particulier où le motif filtrant mis en oeuvre est un dérivé de 3-benzylidène camphre, il convient en outre de noter que ce motif filtrant peut éventuellement posséder deux chainons de raccordement (ceci est effectivement possible lorsque X¹ représente un radical divalent -Y- et lorsque, simultanément, X² ou X³ représente un radical Z porteur de ce même radical divalent -Y-) et être ainsi relié à deux chaines siliconées ou à deux motifs sylilés différents.

Dans le cas d'un motif filtrant de type benzotriazole (formule 4.2) ou benzimidazole (formule 4.3) ou benzophénone (formule 4.4), il ne peut y avoir qu'un seul chainon de raccordement -Y- ; ce dernier est situé à l'une des positions occupées par les symboles X⁴ et X⁵ dans le cas des benzotriazoles, et à des positions imposées bien déterminées dans les autres derniers cas.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, R' et B préférés selon l'invention sont les radicaux méthyle, éthyle, propyle, n-butyle, n-octyle et éthyl-2 hexyle. Encore plus préférentiellement, les radicaux R, R' et B sont tous des radicaux méthyle. Les halogènes peuvent être, quant à eux, notamment choisis parmi chlore, fluor et brome, et sont de préférence le chlore. Les radicaux alcoxy peuvent être choisis notamment parmi les radicaux méthoxy, éthoxy, n-propoxy, isopropoxy, n-butoxy et isobutoxy.

Parmi les composés de formules (1) à (3) ci-dessus, on préfère mettre en oeuvre ceux répondant à la formule (1) ou à la formule (2), c'est à dire des diorganosiloxanes à chaine courte, linéaire ou cyclique.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble (sauf cas d'exclusions mutuelles prévues précédemment pour les trois symboles X¹, X² et X³ lorsque le motif filtrant est un dérivé du 3-benzylidène camphre, ou pour les deux symboles X⁴ et X⁵ lorsque le motif filtrant est une benzotriazole), des caractéristiques suivantes :
- R est alkyle et encore plus préférentiellement est méthyle,
- B est alkyle et encore plus préférentiellement méthyle (cas des composés linéaires de formule (1)),
- r est compris entre 0 et 3 inclusivement ; s est compris entre 0 et 3 inclusivement (cas des composés linéaires de formule (1)),
- t+u est compris entre 3 et 5 (cas des composés cycliques de formule (2)),

- R¹ est H,
- R² est H ou méthyle,
- p est compris inclusivement entre 1 et 3,

- X¹ est H ou -Y-,
- X² est H, méthyle, méthoxy, -Y- ou Z,
- X³ est H ou -Y- ;.

- X⁴ est terbutyle ou -Y- ;
- X⁵ est H ou -Y-,
- R³ est méthyle ou terbutyle.

Les composés de formule (1) à (3) préférés dans le cadre de la présente invention sont ceux qui présentent des motifs filtrants A choisis parmi les dérivés de 3-benzylidène camphre répondant à la formule (4.1) donnée ci-avant.

Pour préparer les filtres siliconés de formule (1) et (2), on peut procéder classiquement (voie 1) en mettant en oeuvre une réaction d'hydrosylilation : 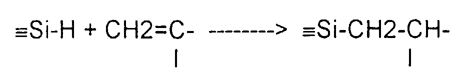 à partir de la silicone correspondante dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709.

Ce dérivé à SiH peut être donc représenté, selon les cas, soit par la formule (1bis) suivante : 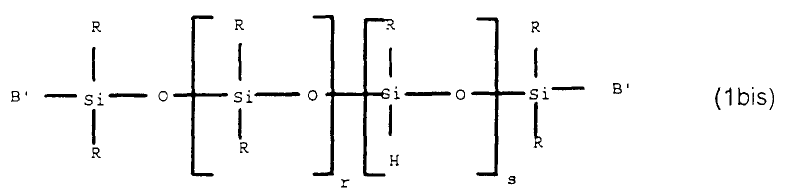 dans laquelle R, r et s ont la signification donnée ci-dessus pour la formule (1 ) et les radicaux B', identiques ou différents, sont choisis parmi les radicaux R et un atome d'hydrogène,
soit par la formule (2bis) suivante : 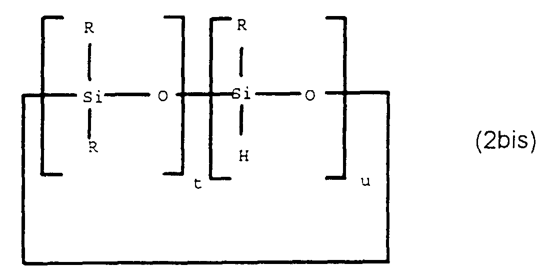 dans laquelle R, t et u ont la signification donnée ci-dessus pour la formule (2).

Sur ce dérivé à SiH de formules (1bis) ou (2bis), on effectue donc une réaction d'hydrosilylation classique, en présence d'une quantité catalytiquement efficace d'un catalyseur au platine, qui est opérée, selon les cas (i.e. selon la nature du motif filtrant à greffer) :
- soit sur un dérivé organique de 3-benzylidène camphre de formule (4.1bis) suivante : 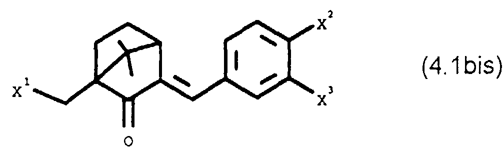 dans laquelle X¹, X² et X³ ont la même signification que celle donnée ci-avant pour la formule (4.1), à cette seule différence près que l'un de ces trois radicaux, au lieu de représenter un radical divalent saturé -Y- de formule (5) définie ci-avant, représente ici le radical monovalent homologue insaturé correspondant -Y', de formule (5bis) suivante : 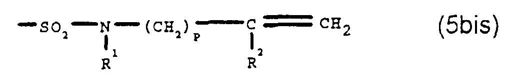 dans laquelle R¹, R² et p ont la même signification qu'à la formule (5);
- soit sur un dérivé organique de benzotriazole de formule (4.2bis) suivante : 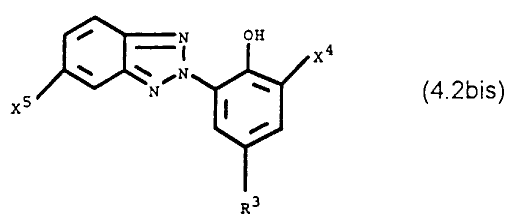 dans laquelle X⁴ et X⁵ ont la même signification que celle donnée ci-avant pour la formule (4.2), à cette seule différence près que l'un de ces deux radicaux, au lieu de représenter un radical divalent saturé -Y- de formule (5) définie ci-avant, représente ici le radical monovalent homologue insaturé correspondant -Y', de formule (5bis) donnée ci-dessus ;
- soit sur un dérivé organique de benzimidazole de formule (4.3bis) suivante : 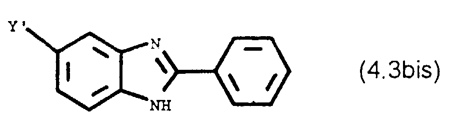 dans laquelle Y' représente le radical monovalent insaturé de formule (5bis) donnée ci-dessus, homologue du diradical saturé -Y- de la formule (4.3) ;
- soit un dérivé organique de benzophénone de formule (4.4bis) suivante : 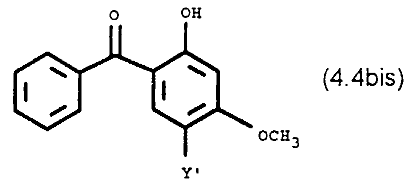 dans laquelle Y' représente le radical monovalent insaturé de formule (5bis) donnée ci-dessus, homologue du diradical saturé -Y- de la formule (4.4).

Parmi les composés de formule (4.1bis) ci-dessus rentrant particulièrement bien dans le cadre de la présente invention, on peut notamment citer :
a) le N-Allyl-4-(4,7,7-trimethyl-3-oxo-bicyclo[2.2.1]hept-2-ylidenemethyl)-benzenesulfonamide
b) le N-Allyl-C-[3-(4-methoxy-benzylidene)-7,7-dimethyl-2-oxo-bicydo[2.2.1] hept-1-yl]-methanesulfonamide
c) le N-Allyl-C-(3-benzylidene-7,7-dimethyl-1-oxo-bicyclo[2,2,1]hept-1-yl)-methanesulfonamide
d) le N-Allyl-C-(3(benzo[1,3]dioxol-5-ylmethylene)-7,7-dimethyl-2-oxo-bicyclo[2.2.1]hept-1-yl)-methanesulfonamide
e) le N-(2-Methyl-allyl)-4-(4,7,7-trimethyl-3-oxo-bicyclo[2.2.1]hept-2-ylidenemethyl)-benzenesulfonamide.

Les produits de formules (4.1bis) à (4.4bis) ci-dessus peuvent, quant à eux, être classiquement obtenus (voir notamment, à cet égard, FR-A- 2 529 887 pour ce qui concerne les composés de formule 4.1bis) en faisant réagir (i) l'amine insaturée correspondante de formule 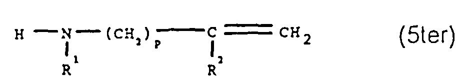 dans laquelle R¹, R² et p ont la même signification que dans la formule (5)
avec (ii) le chlorure de sulfonyle du motif filtrant que l'on désire greffer, à savoir donc :
- soit un chlorure de sulfonyle d'un dérivé organique de 3-benzylidène camphre choisi parmi ceux de formule (4.1ter) suivante : 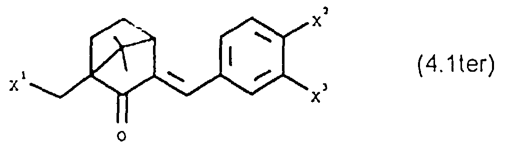 dans laquelle X¹, X² et X³ ont la même signification que celle donnée ci-avant pour la formule (4.1), à cette seule différence près que l'un de ces trois radicaux, au lieu de représenter un radical divalent -Y- de formule (5) définie ci-avant, représente ici le radical monovalent -SO₂Cl ;
- soit un chlorure de sulfonyle d'un dérivé organique de benzotriazole choisi parmi ceux de formule (4.2ter) suivante : 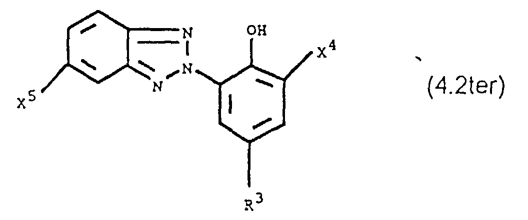 dans laquelle X⁴ et X⁵ ont la même signification que celle donnée ci-avant pour la formule (4.2), à cette seule différence près que l'un de ces deux radicaux, au lieu de représenter un radical divalent saturé -Y- de formule (5) définie ci-avant, représente ici le radical monovalent -SO₂Cl ;
- soit un chlorure de sulfonyle de benzimidazole de formule (4.3ter) suivante 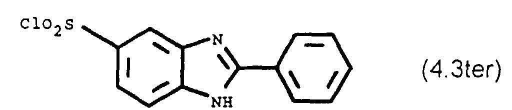
- soit un chlorure de sulfonyle de benzophénone de formule (4.4ter) suivante

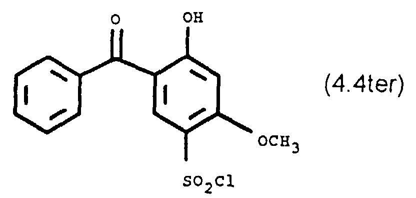

Tous les chlorures de sulfonyle ci-dessus peuvent eux-mêmes être classiquement obtenus par réaction, dans un solvant comme le DMF, entre (i) les sulfonates de sodium correspondants (c'est à dire des composés de formules 4.1bis, 4.2bis, 4.3bis et 4.4bis dans lesquels le radical -Y' désigne ici -SO₃Na) et (ii) du chlorure de thionyle (voir notamment à cet égard la demande FR-A- 2 529 887 précitée).

Les catalyseurs au platine utilisés pour réaliser la réaction d'hydrosylilation entre les composés de formule (1bis) ou (2bis) ci-dessus avec les composés de formules (4.1bis) à (4.4bis) ci-dessus sont bien connus et amplement décrits dans la littérature. On peut en particulier citer les complexes du platine et d'un produit organique décrit dans les brevets américains US-A- 3 159 601, US-A- 3 159 602, US-A- 3 220 972 et les demandes de brevets européens EP-A- 0 057 459, EP-A- 0 188978 et EP-A- 0 190 530 et les complexes du platine et d'organopolysiloxane vinylé décrits dans les brevets américains US-A- 3 419 593, US-A- 3 377 432 et US-A- 3 814 730. Pour faire réagir les composés de formule (1bis) ou (2bis) avec les composés de formules (4.1bis) à (4.4bis), on utilise généralement une quantité de catalyseur au platine, calculée en poids de platine métal, comprise entre 5 et 600 ppm, de préférence entre 10 et 200 ppm, basée sur le poids de composés de formule (1bis) ou (2bis). La réaction d'hydrosylilation peut avoir lieu en masse ou au sein d'un solvant organique volatil tel que le toluène, l'heptane, le xylène, le tétrahydrofuranne et le tétrachloréthylène. Il est généralement souhaitable de chauffer le mélange réactionnel à une température comprise entre 60 et 120°C pendant le temps pour que la réaction soit complète. On peut ajouter goutte à goutte le composé de formule (1bis) ou (2bis) sur le composé de formule (4.1bis), (4.2bis), (4.3bis) ou (4.4bis) en solution dans un solvant organique contenant le catalyseur. On peut aussi ajouter simultanément le composé de formule (1bis) ou (2bis) et le composé de formule (4.1bis) (respectivement (4.2bis) à (4.4bis)) à une suspension de catalyseur dans un solvant organique. Il est recommandé de vérifier que la réaction est complète en dosant les SiH résiduels par la potasse alcoolique, puis on élimine le solvant, par exemple par distillation sous pression réduite. L'huile brute obtenue peut être purifiée, par exemple par passage sur une colonne absorbante de silice.

Concernant la préparation des filtres de type triorganosilanes de formule (3) donnée précédemment, on peut procéder comme indiqué ci-dessus, toujours par réaction d'hydrosylilation, entre cette fois un silane de départ de formule (R')₃Si-H (formule (3bis), dans laquelle R' a la même signification que pour le composé de formule (3), et un dérivé organique choisi parmi (selon le motif filtrant désiré pour le produit final) un dérivé organique de 3-benzylidène camphre de formule (4.1bis) définie ci-avant, un dérivé organique de benzotriazole de formule (4.2bis) définie ci-avant, un dérivé organique de benzimidazole de formule (4.3bis) définie ci-avant, et un dérivé organique de benzophénone de formule (4.4bis) définie ci-avant.

Une autre voie de synthèse possible (voie 2) convenant à la préparation des filtres siliconés de formules (1) et (2) consiste à partir des dérivés correspondant respectivement à la formule (1) ou à la formule (2) dans laquelle tous les radicaux A sont remplacés par le radical de formule (5quater) suivante : 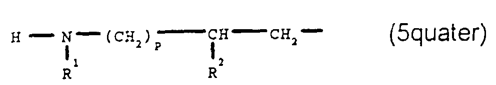 dans laquelle R¹, R² et p ont la même signification que dans la formule (5).

Les radicaux de formule (5quater) peuvent être présents dans la chaine et/ou aux extrémités de la chaine siliconée. Ces dérivés amino siloxanes de départ peuvent ainsi être représentés soit par la formule (1ter) suivante (dérivé amino siloxane linéaire) : 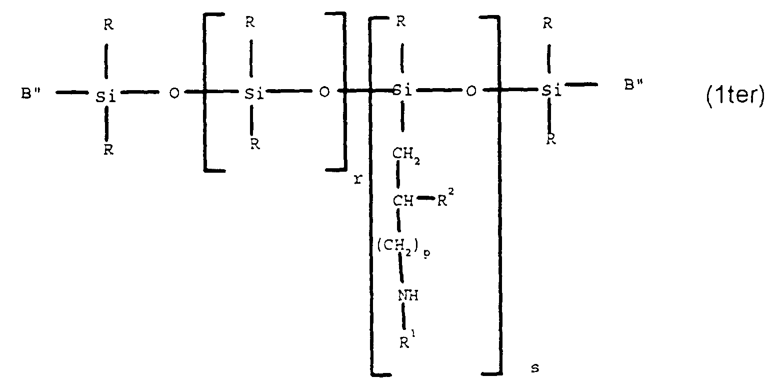 dans laquelle R, r et s ont la signification donnée ci-dessus pour la formule (1) et les radicaux B", identiques ou différents, sont choisis parmi les radicaux R et le radical de formule (5quater),
soit par la formule (2ter) suivante (dérivé amino siloxane cyclique) : 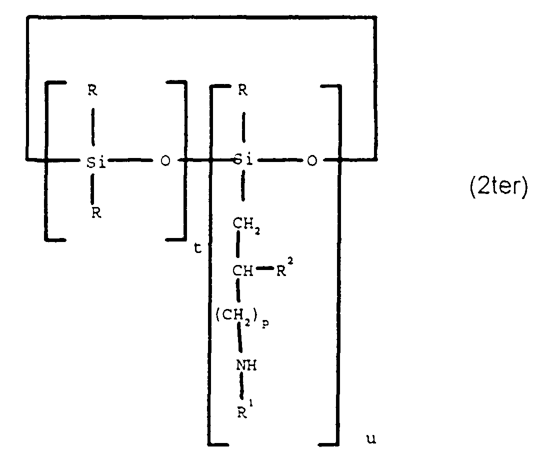 dans laquelle R, t et u ont la signification donnée ci-dessus pour la formules (2).

Les dérivés amino siloxanes de formule (1ter) ou (2ter) ci-dessus sont des produits bien connus dans l'industrie des silicones et sont généralement disponibles dans le commerce. Ils sont en outre notamment décrits dans la demande de brevet DE-A 3 702 631, pour ce qui concerne les silicones à chaines courtes.

Sur ces dérivés amino siloxanes, on fait alors réagir le chlorure de sulfonyle du motif filtrant que l'on désire greffer, à savoir donc :
- soit un chlorure de sulfonyle d'un dérivé organique de 3-benzylidène camphre choisi parmi ceux de formule (4.1ter) définie ci-avant ;
- soit un chlorure de sulfonyle d'un dérivé organique de benzotriazole choisi parmi ceux de formule (4.2ter) définie ci-avant ;
- soit un chlorure de sulfonyle de benzimidazole de formule (4.3ter) définie ci-avant ;
- soit un chlorure de sulfonyle de benzophénone de formule (4.4ter) définie ci-avant.

Par rapport aux silicones filtres de l'art antérieur, et en particulier à celles décrites dans les demandes de brevet EP-A- 0 392 883 et EP-A- 0 335 777 précitées, les silicones filtres selon l'invention présentent donc une ou plusieurs différences structurelles essentielles, à l'origine de leurs propriétés remarquables, et notamment : les chaines siliconées, sur lesquelles sont greffés les motifs filtrants sélectionnés, sont tout d'abord beaucoup plus courtes ; ensuite, le motif filtrant sélectionné porte toujours une fonction sulfonamide.

Comme indiqué précédemment, les composés de formules (1) à (3) ci-dessus présentent un excellent pouvoir filtrant intrinsèque à l'égard du rayonnement ultraviolet (UV-A et/ou UV-B, selon la structure du produit). En mélangeant des produits de structure différente, c'est à dire plus précisément en mélangeant des produits conformes à l'invention présentant une activité purement UV-A avec des produits conformes à l'invention présentant une activité purement UV-B, il est ainsi possible de disposer d'une composition qui présentera globalement une activité filtrante dans toute la gamme des UV nocifs (UV-A + UV-B), ce qui constitue un avantage important. En outre, de par leur caractère fortement liposoluble, les composés de formule (1) à (3) ci-dessus peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. Enfin, leurs propriétés cosmétiques sont très bonnes, à savoir en particulier que ces produits, par rapport aux silicones filtres de l'art antérieur, sont moins collantes et apportent plus de douceur.

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) à (3) définies ci-avant.

Les composés de formules (1) à (3) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.
Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.
Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de la peau et cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2) ou (3) tel que définie ci-avant.

Les exemples qui suivent illustrent l'invention sans toutefois en limiter la portée.

### EXEMPLE 1

Cet exemple illuste la préparation (selon la voie 1) du N-3-[1,3,3,3-tetramethyl-1-[(trimethylsilyl)oxy]disiloxanyl]propyle-4-(4,7,7-trimethyl-3-oxo-bicyclo[2.2.1]hept-2-ylidenemethyl)-benzenesulfonamide, c'est à dire d'un composé conforme à l'invention répondant à la formule : 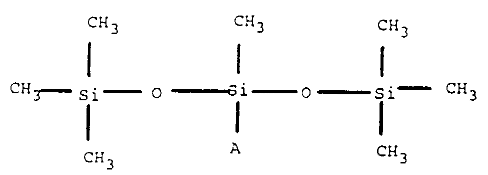 dans laquelle A représente : 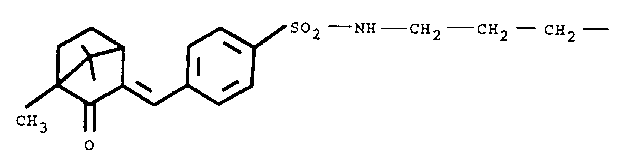 (ce produit correspond à un composé de formule (1) pour lequel R = B = CH₃ ; r = 0 ; s = 1 ; X¹ = X³ = H ; R¹ = R² = H ; p = 1).

### a) Première étape :

Dans un réacteur de 500 ml, on introduit 34,2 g de sel de sodium de l'acide 4-(4,7,7-Trimethyl-3-oxo-bicyclo[2.2.1]hept-2-ylidenemethyl)-benzenesulfonique et 200 ml de diméthylformamide. Puis, on introduit goutte à goutte 13 g de chlorure de thionyle. Le mélange réactionnel est ensuite agité pendant 2 heures à température ambiante. L'allylamine (6,3 g) puis la triéthylamine (11,1 g) sont ensuite ajoutés goutte à goutte et on maintient l'agitation pendant 3 heures. Le mélange réactionnel est versé dans 300 ml d'eau. Le solide obtenu est essoré puis séché et, après purification, on récupère 21,4 g de N-Allyl-4-(4,7,7-trimethyl-3-oxo-bicyclo[2.2.1]hept-2-ylidenemethyl)-benzenesulfonamide,de caractéristiques suivantes :
Poudre blanche
Pf : 131°C

| Analyse élémentaire : | | | | |
|---|---|---|---|---|
| théorie : | C67,09 | H7,04 | N3,66 | S8,88 |
| trouvé : | C66,82 | H7,01 | N3,90 | S8,92 |

### b) Deuxième étape :

Dans un réacteur, on charge 17,97 g du produit obtenu précédemment et 50 ml de toluène. Le mélange est porté à 80°C sous azote. On ajoute le catalyseur d'hydrosylilation (complexe à 3-3,5% de Pt dans du cyclovinylméthylsiloxane de Hüls Petrarch PC085 : 100 µl) puis 11,24 g d'heptaméthyltrisiloxane.

Après 4 heures à 80°C sous azote, on concentre le milieu réactionnel et on effectue une chromatographie sur Silice sous pression (éluant : heptane/EtOAc 97:3). On obtient alors 21,8 g du produit final désiré, de caractéristiques suivantes:
Poudre blanche
Pf : 73°C

| Analyse élémentaire : | | | | | |
|---|---|---|---|---|---|
| théorie : | C55,72 | H8,14 | N2,41 | S5,51 | Si14,48 |
| trouvé : | C55,88 | H8,09 | N2,62 | S5,49 | Si14,28 |

Les caractéristiques en absorption UV (mesurée dans l'éthanol) de ce produit sont les suivantes :

| | |
|---|---|
| λₘₐₓ : 295 nm | εₘₐₓ : 28 900 |

Ce produit peut donc être utilisé très efficacement à titre de filtre solaire actif dans l'UV- B.

### EXEMPLE 2

En suivant le même mode opératoire que celui donné à l'exemple 1, on a préparé quatre autres composés conformes à l'invention (composés A à D), rentrant tous dans le cadre de la formule générale (1) et ayant le même squelette siliconé suivant : 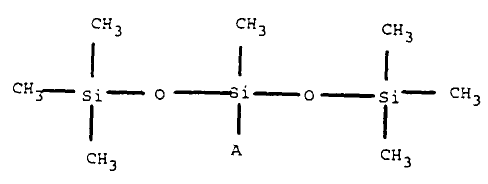 mais présentant à chaque fois un motif filtrant A de nature différente.

Les résultats sont rassemblés dans le tableau I.

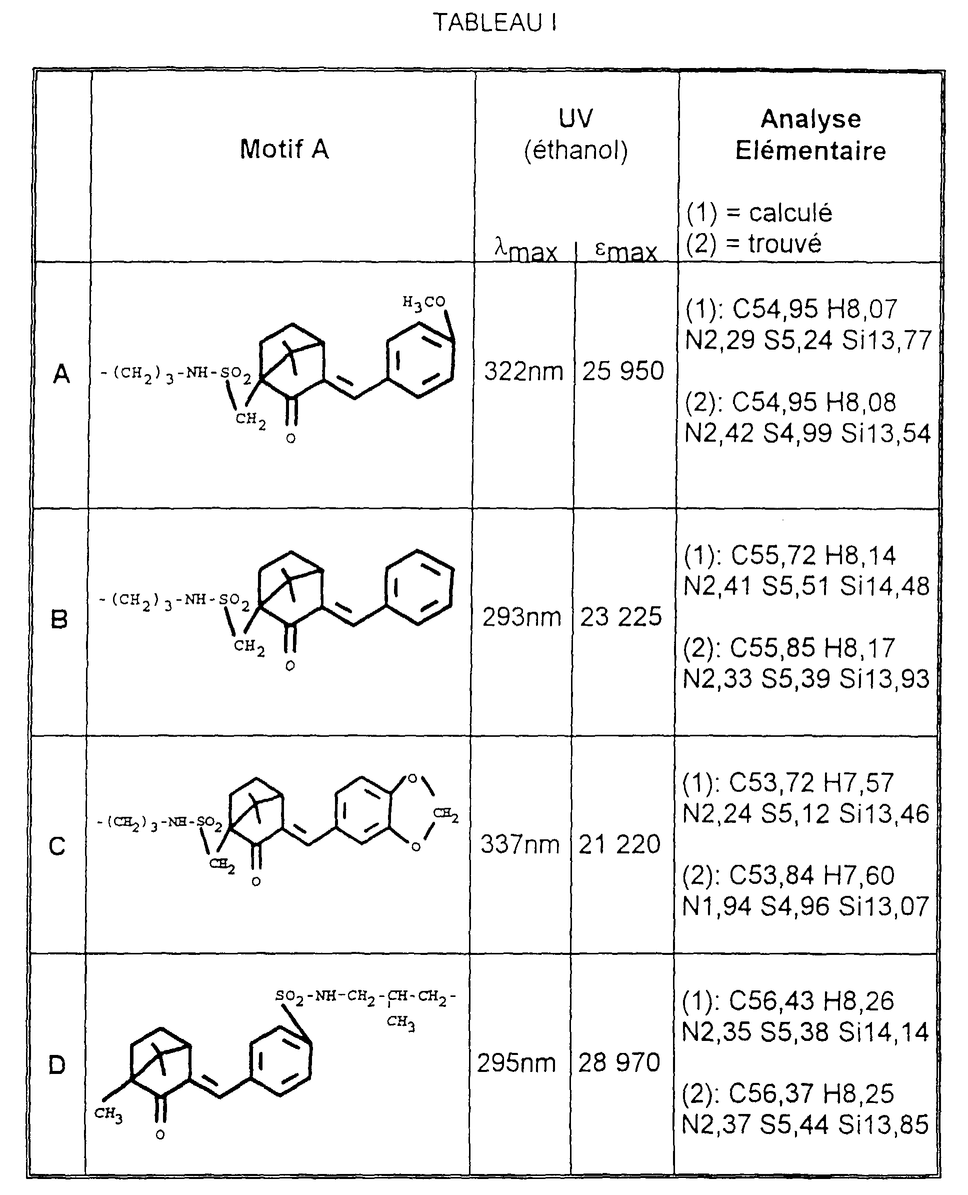

Les composés A, B, C et D ci-dessus peuvent donc être utilisés très efficacement à titre de filtre solaire actif dans, respectivement, l'UV- A, l'UV- B, l'UV- A et l'UV-B.

### EXEMPLE 3

Cet exemple illuste la préparation (selon la voie 2) du Bis N{3-[1,3,3,3-tetramethyl-1-[(trimethylsilyl)oxy]disiloxanyl]propyle}-3,3'-terephtalylidene-10,10'-dicamphosulfonamide, c'est à dire d'un composé conforme à l'invention répondant à la formule : 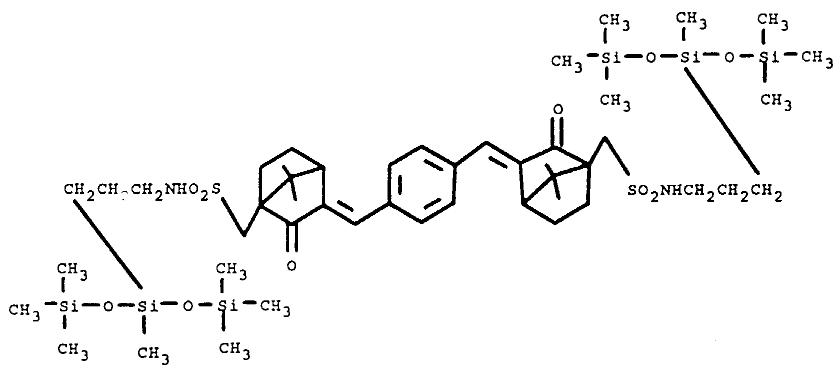 (ce produit correspond à un composé de formule (1) pour lequel R = B = CH₃ ; r = 0 ; s = 1 ; X¹ = -Y- avec R¹ = R² = H et p = 1 ; X² = Z ; X³ = H).

Dans un réacteur, on charge 4 g du sel disodique de l'acide 3,3'-térephtalylidène-10,10'-dicamphosulfonique et 16 ml de diméthylformamide. Puis, on y ajoute goutte à goutte 1,1 ml de chlorure de thionyle et on maintient l'agitation pendant une demi-heure. Ce mélange hétérogène est ajouté portion par portion à un mélange de triéthylamine (3,04 g) et d'amino-1[1,3,3,3-tetramethyl-1-[(trimethylsilyl)oxy]disiloxanyl]-3-propane(4,2 g). On maintient 3 heures à température ambiante, puis on verse sur 100 ml d'eau glacée, et enfin on extrait au dichlorométhane. La phase organique est séchée sur sulfate de sodium et concentrée. Après chromatographie sur Silice (éluant : CH₂Cl₂), on obtient alors 3 g du produit final désiré, de caractéristiques suivantes:
Poudre blanche
Pf : 176-177°C

| Analyse élémentaire : | | | | | |
|---|---|---|---|---|---|
| théorie : | C52,23 | H8,00 | N2,65 | S6,06 | Si15,93 |
| trouvé : | C52,92 | H8,12 | N2,43 | S5,94 | Si15,37 |

Les caractéristiques en absorption UV (mesurée dans CHCl3) de ce produit sont les suivantes :

| | |
|---|---|
| λₘₐₓ : 344 nm | εₘₐₓ : 47 100 |

Ce produit peut donc être utilisé très efficacement à titre de filtre solaire actif dans l'UV- A.

### EXEMPLE 4

Cet exemple illustre la préparation (selon la voie 2) du 2-phényl-1H-benzoimidazole-5-{N-3-[1,3,3,3-tétraméthyl-1-[(triméthylsilyl)oxy]disiloxanyl]propyle}-sulfonamide, c'est à dire d'un composé conforme à l'invention répondant à la formule : 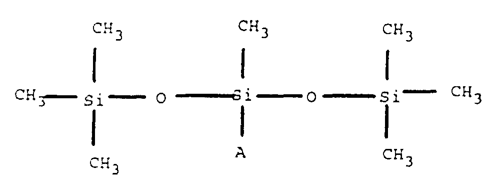 dans laquelle A représente : 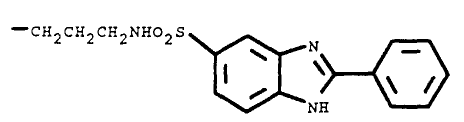 (ce produit correspond à un composé de formule (1) pour lequel R = B = CH₃ ; r = 0 ; s = 1 ; R¹ = R² = H et p = 1)

Dans un réacteur, on charge 100 ml de dichlorométhane sec, 60 ml de triéthylamine et 67 g d'heptaméthylaminopropyltrisiloxane (produit correspondant à un composé de formule (1ter) dans laquelle R = B'' = CH₃ ; r = 0 ; s = 1 ; R¹ = R² = H ; p = 1). A température ambiante, on ajoute à ce mélange, par portion et en une demi-heure, 23,4 g du chlorure de l'acide 2-phényl-1H-benzoimidazole-5-sulfonique. Le mélange hétérogène obtenu est ensuite chauffé à 60°C pendant 3 heures. Après refroidissement, le mélange réactionnel est versé dans 300 ml d'eau. Après extraction au dichlorométhane et purification par chromatographie sur silice (éluant : heptane/CH2Cl2 50:50), on obtient 8 g du produit final désiré, de caractéristiques suivantes :
Poudre blanche
Pf : 169-170°C

Les caractéristiques en absorption UV (mesurée dans l'éthanol) de ce produit sont les suivantes :

| | |
|---|---|
| λₘₐₓ : 305 nm | εₘₐₓ : 29 500 |
| λₘₐₓ : 317 nm | εₘₐₓ : 19 675 |

Ce produit peut donc être utilisé très efficacement à titre de filtre solaire actif dans l'UV- B.

### EXEMPLE 5

En suivant le même mode opératoire que celui donné à l'exemple 4, on a préparé un autre composé conforme à l'invention répondant à la formule suivante : 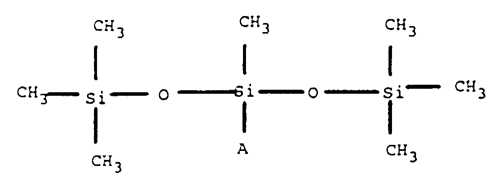 dans laquelle A représente : 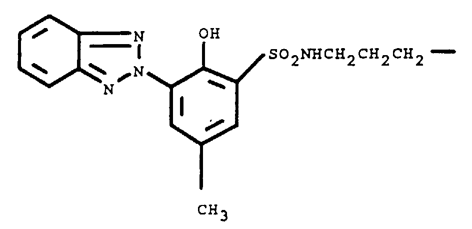 (ce produit correspond à un composé de formule (1) pour lequel R = B = CH₃ ; r = 0 ; s = 1 ; X⁴ = -Y- avec R¹ = R² = H et p = 1 ; X⁵ = H ; R³ = méthyle).

Les caractéristiques en absorption UV (mesurée dans l'éthanol) de ce produit sont les suivantes :

| | |
|---|---|
| λₘₐₓ : 294 nm | εₘₐₓ : 12 100 |
| λₘₐₓ : 325 nm | εₘₐₓ : 7 300 |

Ce produit peut donc être utilisé très efficacement à titre de filtre solaire actif dans l'UV-B et l'UV-A.

### EXEMPLE 6

On illustre ici une formulation concrète de composition cosmétique antisolaire conforme à l'invention, à savoir une crème antisolaire.
- Composé de l'exemple 1 5 g
- Mélange d'alcool cétylstéarylique et d'alcool cétylstéarylique oxyéthyléné à 33 moles d'OE ("SINNOVAX AO®" de chez HENKEL) 7 g
- Mélange de mono et distéarate de glycérol non autoémulsifiable 2 g
- Alcool cétylique 1,5 g
- Benzoate d'alcools en C₁₂-C₁₅ ("FINSOLV TN®" de chez WITCO) 20 g
- Polydiméthylsiloxane 1,5 g
- Glycérine 17,5 g
- Parfum, conservateur qs
- Eau qsp 100 g

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

## Revendications

1. Nouveaux composés de formules (1) à (3) suivantes : 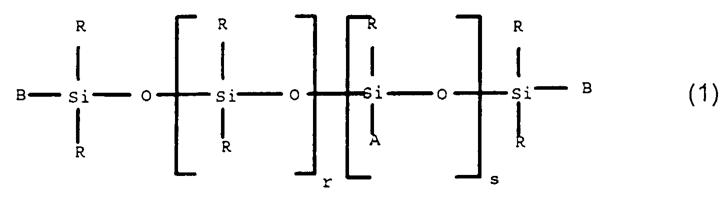 ou 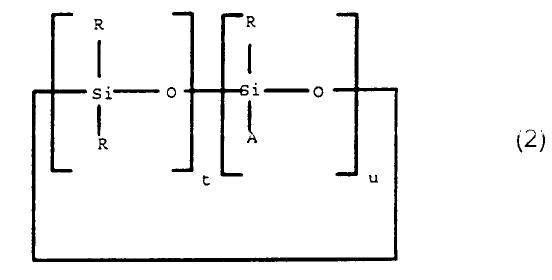 ou
A-Si(R')₃ (3)
formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical lié directement à un atome de silicium, ledit radical A répondant à l'une des formules (4.1 à (4.4) suivantes : 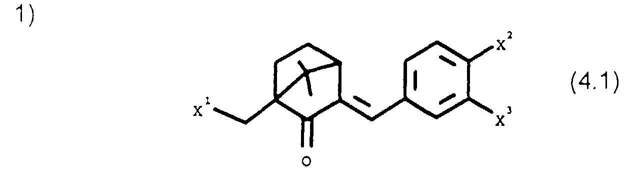 formule (4.1) dans laquelle :
∗ **X**^{**1**} représente un atome d'hydrogène, ou un radical divalent -Y- de formule (5) suivante : 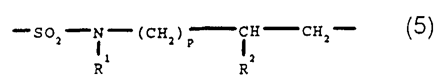 formule (5) dans laquelle :
- R¹ désigne un atome d'hydrogène, un radical alkyle ou hydroxyalkyle en C₁-C₄,
- R² représente un atome d'hydrogène, un radical alkyle en C₁-C₆ ou alcoxy en C₁-C₆,
- p est un nombre entier compris entre 1 et 10 inclusivement,
- le groupement -CH₂- terminal est directement lié à un atome de silicium ;
∗ **X**^{**2**} **et X**^{**3**}, identiques ou différents, représentent un atome d'hydrogène ou d'halogène, un radical alkyle ou alcoxy en C₁-C₄, un radical divalent -Y-, ou un radical Z de formule (6) suivante : 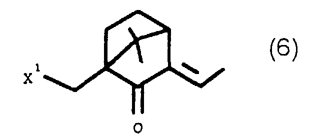 ou encore peuvent former ensemble un groupement alkylidène dioxy dans lequel le groupe alkylidène contient 1 ou 2 atomes de carbone;
étant entendu que dans cette formule (4.1) :
- l'un des trois symboles X¹, X² et X³ doit nécessairement représenter un radical divalent -Y-, et aucun des deux autres symboles restants ne peut alors représenter un radical divalent -Y-,
- si X¹ représente un atome d'hydrogène, alors X² et X³ sont nécessairement différents et aucun ne peut représenter un radical Z,
- si X¹ représente un radical divalent -Y-, alors X² et X³ ne peuvent pas simultanément représenter un radical Z,
- si X¹ désigne un atome d'hydrogène et X³ représente un radical divalent -Y-, alors X² est de préférence différent d'un atome d'hydrogène ; 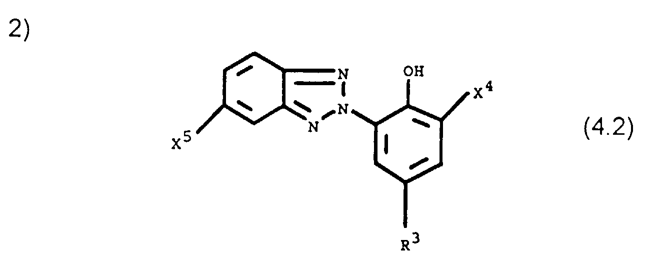 formule (4.2) dans laquelle :
∗ **X**^{**4**} représente un atome d'hydrogène, un radical alkyle en C₁-C₈ ou un radical divalent -Y-,
∗ **X**^{**5**} représente un atome d'hydrogène ou d'halogène, un radical alkyle ou alcoxy en C₁-C₄ ou un radical divalent -Y-,
∗ **R**^{**3**} représente un radical alkyle en C₁-C₈,
étant entendu que dans cette formule (4.2) l'un des deux symboles X⁴, X⁵ doit nécessairement représenter un radical divalent -Y-, le symbole restant ne pouvant pas représenter un radical divalent -Y- ;

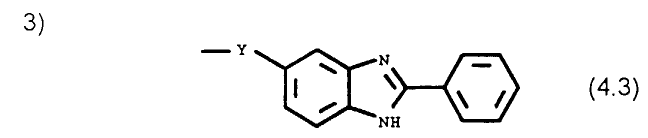

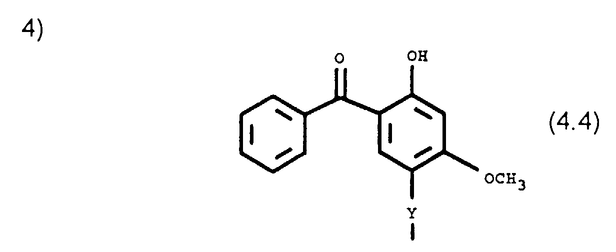

2. Nouveaux composés selon la revendication 1, répondant à la formule (1 ) ou à la formule (2), caractérisés par le fait que les radicaux R sont des radicaux alkyle.

3. Nouveaux composés selon la revendication 2, caractérisés par le fait que les radicaux R sont des radicaux méthyle, éthyle, propyle, n-butyle, n-octyle ou éthyl-2 hexyle.

4. Nouveaux composés selon la revendication 3, caractérisés par le fait que les radicaux R sont des radicaux méthyle.

5. Nouveaux composés selon l'une quelconque des revendications précédentes, répondant à la formule (1), caractérisés par le fait que les radicaux B sont des radicaux alkyle.

6. Nouveaux composés selon la revendication 5, caractérisés par le fait que les radicaux B sont des radicaux méthyle, éthyle, propyle, n-butyle, n-octyle ou éthyl-2 hexyle.

7. Nouveaux composés selon la revendication 6, caractérisés par le fait que les radicaux B sont radicaux méthyle.

8. Nouveaux composés selon l'une quelconque des revendications précédentes, répondant à la formule (1), caractérisés par le fait que r est compris entre 0 et 3 inclusivement, et s est compris entre 0 et 3 inclusivement.

9. Nouveaux composés selon l'une quelconque des revendications 1 à 4, répondant à la formule (2), caractérisés par le fait que t + u est compris entre 3 et 5 inclusivement.

10. Nouveaux composés selon la revendication 1, répondant à la formule (3), caractérisés par le fait que les radicaux R' sont des radicaux alkyle choisis parmi les radicaux méthyle, éthyle, propyle, n-butyle, n-octyle ou éthyl-2 hexyle.

11. Nouveaux composés selon la revendication 10, caractérisés par le fait que les radicaux R' sont des radicaux méthyle.

12. Nouveaux composés selon l'une quelconque des revendications précédentes, caractérisés par le fait que dans le radical divalent -Y-, p est compris inclusivement entre 1 et 3.

13. Nouveaux composés selon l'une quelconque des revendications précédentes, caractérisés par le fait que dans le radical divalent -Y-, R¹ représente l'hydrogène.

14. Nouveaux composés selon l'une quelconque des revendications précédentes, caractérisés par le fait que dans le radical divalent -Y-, R² représente l'hydrogène ou un radical méthyle.

15. Nouveaux composés selon l'une quelconque des revendications précédentes, caractérisés par le fait que A répond à la formule (4.1).

16. Nouveaux composés selon la revendication 15, caractérisés par le fait que X² est hydrogène, méthyle, méthoxy ou -Y-.

17. Nouveaux composés selon l'une quelconque des revendications 15 ou 16, caractérisés par le fait que X³ est hydrogène ou -Y-.

18. Nouveaux composés selon l'une quelconque des revendications 1 à 14, caractérisés par le fait que A répond à la formule (4.2).

19. Nouveaux composés selon la revendication 18, caractérisés par le fait que X⁴ est terbutyle ou -Y-.

20. Nouveaux composés selon l'une quelconque des revendications 18 ou 19, caractérisés par le fait que X⁵ est hydrogène ou -Y-.

21. Nouveaux composés selon l'une quelconque des revendications 18 à 20, caractérisés par le fait que R³ est méthyle ou terbutyle.

22. Nouveaux composés selon l'une quelconque des revendications 1 à 14, caractérisés par le fait que A répond à la formule (4.3).

23. Nouveaux composés selon l'une quelconque des revendications 1 à 14, caractérisés par le fait que A répond à la formule (4.4).

24. Utilisation des composés de formule (1)-(3) tels que définis à l'une quelconque des revendications précédentes, à titre de filtres solaires actifs dans le domaine des UV-A et/ou UV-B.

25. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 23.

26. Composition cosmétique selon la revendication 25, caractérisée par le fait que ledit support cosmétiquement acceptable contient au moins une phase grasse ou un solvant organique.

27. Composition cosmétique selon la revendication 26, caractérisée par le fait que ledit support se présente sous la forme d'une émulsion de type huile-dans-eau ou eau-dans-huile, de préférence huile-dans-eau.

28. Composition cosmétique selon l'une des revendications 25 à 27, caractérisée par le fait que la teneur en composé(s) filtrant(s) est comprise entre 0,1 et 20 % en poids par rapport au poids total de la composition.

29. Composition cosmétique selon la revendication 28, caractérisée par le fait que ladite teneur est comprise entre entre 0,5 et 10 % en poids.

30. Procédé de protection de la peau et/ou des cheveux contre le rayonnement ultraviolet, en particulier le rayonnement solaire, caractérisé par le fait qu'il consiste à appliquer sur la peau et/ou les cheveux une quantité efficace d'au moins un composé ou d'au moins une composition tels que définis à l'une quelconque des revendications précédentes.

## Claims

1. Novel compounds of following formulae (1) to (3) : 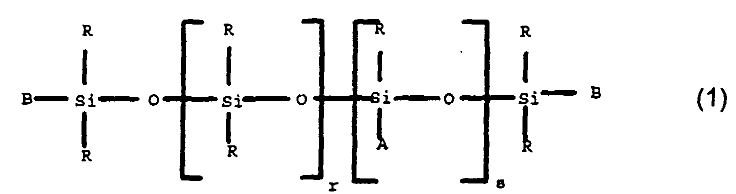 or 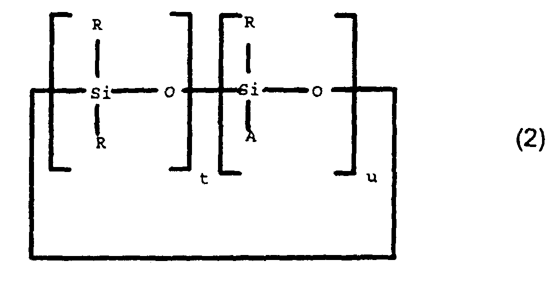 or
A-Si(R')₃ (3)
in which formulae (1) to (3):
- the groups R, which may be identical or different, are chosen from C₁-C₁₀ alkyl, phenyl and 3,3,3-trifluoropropyl radicals, at least 80 %, in numerical terms, of the radicals R being methyl,
- the groups B, which may be identical or different, are chosen from the above radicals R and the radical A defined below,
- the groups R', which may be identical or different, are chosen from C₁-C₈ alkyl radicals, or phenyl radicals,
- r is an integer between 0 and 50 inclusively, and s is an integer between 0 and 20 inclusively, with the condition that if s is zero then at least one of the two symbols B denotes A,
- u is an integer between 1 and 6 inclusively, and t is an integer between 0 and 10 inclusively, it being understood that t + u is equal to or greater than 3,
- and the symbol A denotes a radical directly attached to a silicon atom, the said radical A corresponding to one of the following formulae (4.1) to (4.4): 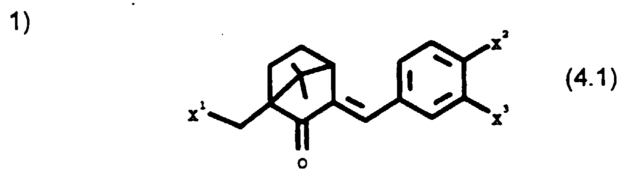 in which formula (4.1):
∗ **X**^{**1**} represents a hydrogen atom or a divalent radical -Y- of following formula (5): 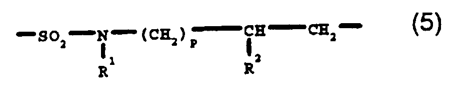 in which formula (5) :
- R¹ denotes a hydrogen atom or a C₁-C₄ alkyl or hydroxyalkyl radical,
- R² represents a hydrogen atom or a C₁-C₆ alkyl or C₁-C₆ alkoxy radical,
- p is an integer between 1 and 10 inclusively,
- the end -CH₂- group is directly attached to a silicon atom;
∗ **X**^{**2**} and **X**^{**3**}, which may be identical or different, represent a hydrogen or halogen atom, a C₁-C₄ alkyl or alkoxy radical, a divalent radical -Y- or a radical Z of following formula (6): 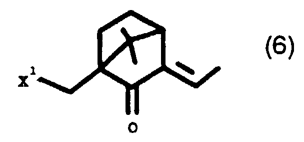 or alternatively may together form an alkylidenedioxy group in which the alkylidene group contains 1 or 2 carbon atoms;
it being understood that in this formula (4.1):
- one of the three symbols X¹, X² and X³ must necessarily represent a divalent radical -Y-, and each of the other two remaining symbols cannot then represent a divalent radical -Y-,
- if X¹ represents a hydrogen atom, X² and X³ are then necessarily different and neither may represent a radical Z,
- if X¹ represents a divalent radical -Y-, X² and X³ cannot then simultaneously represent a radical Z,
- if X¹ denotes a hydrogen atom and X³ represents a divalent radical -Y-, X² is then preferably other than a hydrogen atom; 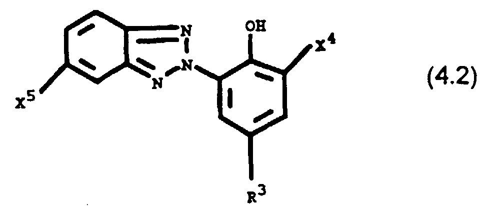 in which formula (4.2):
∗ **X**^{**4**} represents a hydrogen atom, a C₁-C₈ alkyl radical or a divalent radical -Y-,
∗ **X**^{**5**} represents a hydrogen or halogen atom, a C₁-C₄ alkyl or alkoxy radical or a divalent radical -Y-,
∗ **R**^{**3**} represents a C₁-C₈ alkyl radical,
it being understood that, in this formula (4.2), one of the two symbols X⁴ and X⁵ must necessarily represent a divalent radical -Y-, it not being possible for the remaining symbol to represent a divalent radical -Y-,

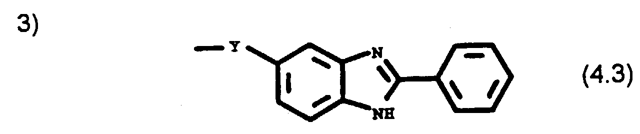

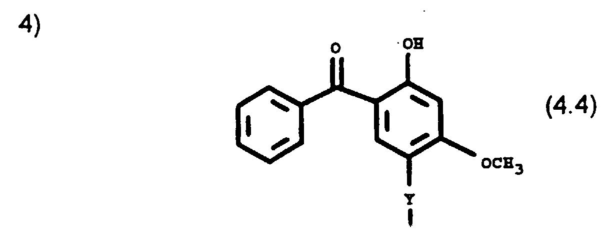

2. Novel compounds according to Claim 1, corresponding to formula (1) or to formula (2), characterized in that the radicals R are alkyl radicals.

3. Novel compounds according to Claim 2, characterized in that the radicals R are methyl, ethyl, propyl, n-butyl, n-octyl or 2-ethylhexyl radicals.

4. Novel compounds according to Claim 3, characterized in that the radicals R are methyl radicals.

5. Novel compounds according to any one of the preceding claims, corresponding to formula (1), characterized in that the radicals B are alkyl radicals.

6. Novel compounds according to Claim 5, characterized in that the radicals B are methyl, ethyl, propyl, n-butyl, n-octyl or 2-ethylhexyl radicals.

7. Novel compounds according to Claim 6, characterized in that the radicals B are methyl radicals.

8. Novel compounds according to any one of the preceding claims, corresponding to formula (1), the preceding claims, corresponding to formula (1), characterized in that r is between 0 and 3 inclusively, and s is between 0 and 3 inclusively.

9. Novel compounds according to any one of Claims 1 to 4, corresponding to formula (2), characterized in that t+u is between 3 and 5 inclusively.

10. Novel compounds according to Claim 1, corresponding to formula (3), characterized in that the radicals R' are alkyl radicals chosen from the methyl, ethyl, propyl, n-butyl, n-octyl and 2-ethylhexyl radicals.

11. Novel compounds according to Claim 10, characterized in that the radicals R' are methyl radicals.

12. Novel compounds according to any one of the preceding claims, characterized in that in the divalent radical -Y-, p is between 1 and 3 inclusively.

13. Novel compounds according to any one of the preceding claims, characterized in that, in the divalent radical -Y-, R¹ represents hydrogen.

14. Novel compounds according to any one of the preceding claims, characterized in that, in the divalent radical -Y-, R² represents hydrogen or a methyl radical.

15. Novel compounds according to any one of the preceding claims, characterized in that A corresponds to formula (4.1).

16. Novel compounds according to Claim 15, characterized in that X² is hydrogen, methyl, methoxy or -Y-.

17. Novel compounds according to either of Claims 15 and 16, characterized in that X³ is hydrogen or -Y-.

18. Novel compounds according to any one of Claims 1 to 14, characterized in that A corresponds to formula (4.2).

19. Novel compounds according to Claim 18, characterized in that X⁴ is tert-butyl or -Y-.

20. Novel compounds according to either of Claims 18 and 19, characterized in that X⁵ is hydrogen or -Y-.

21. Novel compounds according to any one of Claims 18 to 20, characterized in that R³ is methyl or tert-butyl.

22. Novel compounds according to any one of Claims 1 to 14, characterized in that A corresponds to formula (4.3).

23. Novel compounds according to any one of Claims 1 to 14, characterized in that A corresponds to formula (4.4).

24. Use of the compounds of formulae (1)-(3) as defined in any one of the preceding claims, as sunscreens which are active in the UV-A and/or UV-B region.

25. Cosmetic composition, in particular for screening out ultraviolet rays, characterized in that it comprises, in a cosmetically acceptable vehicle, an effective amount of at least one of the compounds defined in any one of Claims 1 to 23.

26. Cosmetic composition according to Claim 25, characterized in that the said cosmetically acceptable vehicle contains at least one fatty phase or one organic solvent.

27. Cosmetic composition according to Claim 26, characterized in that the said vehicle is in the form of an emulsion of oil-in-water or water-in-oil type, preferably of oil-in-water type.

28. Cosmetic composition according to one of Claims 25 to 27, characterized in that the content of screening compound(s) is between 0.1 and 20 % by weight relative to the total weight of the composition.

29. Cosmetic composition according to Claim 28, characterized in that the said content is between 0.5 and 10 % by weight.

30. Process for the protection of the skin and/or the hair against ultraviolet radiation, in particular solar radiation, characterized in that it consists in applying to the skin and/or the hair an effective amount of at least one compound or of at least one composition as are defined in any one of the preceding claims.

## Patentansprüche

1. Neue Verbindungen der folgenden Formeln (1) bis (3): 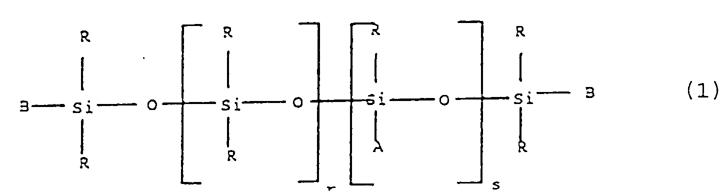 oder 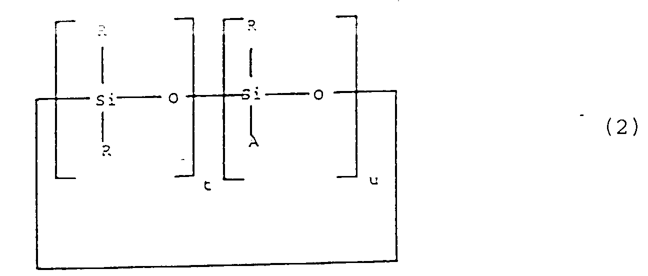 oder
A-Si(R')₃ (3),
wobei in den Formeln (1) bis (3):
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Anzahl der Gruppen R Methyl bedeutet,
- die Gruppen B, die identisch oder voneinander verschieden sind, unter den oben genannten Gruppen R und der nachfolgend definierten Gruppe A ausgewählt sind,
- die Gruppen R', die identisch oder voneinander verschieden sind, unter den C₁₋₈-Alkylgruppen oder Phenyl ausgewählt sind,
- r eine ganze Zahl im Bereich von Null bis einschließlich 50 und s eine ganze Zahl im Bereich von Null bis einschließlich 20 bedeutet, mit der Maßgabe, daß mindestens eine der beiden Gruppen B A bedeutet, wenn s Null ist,
- u eine ganze Zahl im Bereich von 1 bis einschließlich 6 und t eine ganze Zahl im Bereich von Null bis einschließlich 10 bedeutet, mit der Maßgabe, daß t + u mindestens 3 ist, und
- die Gruppe A eine Gruppe bedeutet, die direkt an ein Siliciumatom gebunden ist, wobei die Gruppe A einer der folgenden Formeln (4.1) bis (4.4) entspricht: 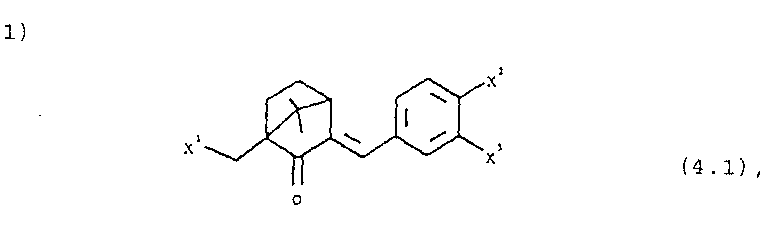 wobei in der Formel (4.1) bedeuten:
X¹ ein Wasserstoffatom oder eine zweiwertige Gruppe -Y- der folgenden Formel (5): 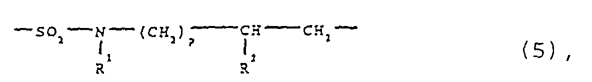 wobei in der Formel (5) bedeuten:
- R¹ Wasserstoff, Alkyl oder Hydroxyalkyl mit 1 bis 4 Kohlenstoffatomen,
- R² Wasserstoff, C₁₋₆-Alkyl oder C₁₋₆-Alkoxy,
- p eine ganze Zahl im Bereich von 1 bis einschließlich 10,
- wobei die -CH₂-Endgruppe direkt an ein Siliciumatom gebunden ist,
X² und X³, die identisch oder voneinander ververschieden sind, Wasserstoff oder Halogen, Alkyl oder Alkoxy mit 1 bis 4 Kohlenstoffatomen, eine zweiwertige Gruppe -Y-, oder eine Gruppe Z der folgenden Formel (6): 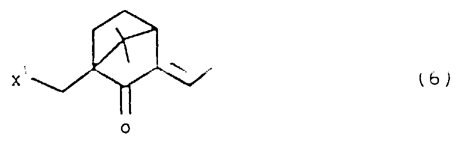 oder X² und X³ können gemeinsam eine Alkylidendioxygruppe bilden, wobei die Alkylidengruppe 1 oder 2 Kohlenstoffatome enthält,
mit der Maßgabe, daß in der Formel (4.1):
- eine der drei Gruppen X¹, X² und X³ zwangsläufig eine zweiwertige Gruppe -Y- bedeuten muß und keine der beiden übrigen anderen Gruppen dann eine zweiwertige Gruppe -Y-bedeuten kann,
- wenn X¹ Wasserstoff bedeutet, X² und X³ zwangsläufig voneinander verschieden sind und beide keine Gruppe Z bedeuten können,
- wenn X¹ eine zweiwertige Gruppe -Y- bedeutet, X² und X³ nicht gleichzeitig eine Gruppe Z bedeuten können,
- wenn X¹ Wasserstoff und X³ eine zweiwertige Gruppe -Y- bedeutet, X² vorzugsweise von Wasserstoff verschieden ist; 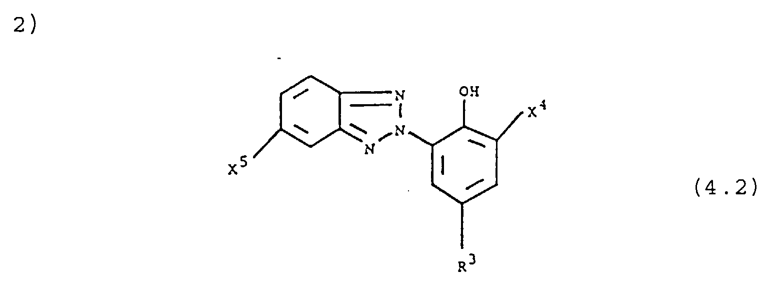 wobei in der Formel (4.2) bedeuten:
X⁴ Wasserstoff, C₁₋₈-Alkyl oder eine zweiwertige Gruppe -Y-,
X⁵ Wasserstoff oder Halogen, Alkyl oder Alkoxy mit 1 bis 4 Kohlenstoffatomen oder eine zweiwertige Gruppe -Y-,
R³ eine C₁₋₈-Alkylgruppe,
mit der Maßgabe, daß in der Formel (4.2) eine der beiden Gruppe X⁴ und X⁵ zwangsläufig eine zweiwertige Gruppe -Y- bedeuten muß, wobei die andere verbleibende Gruppe keine zweiwertige Gruppe -Y- bedeuten kann;

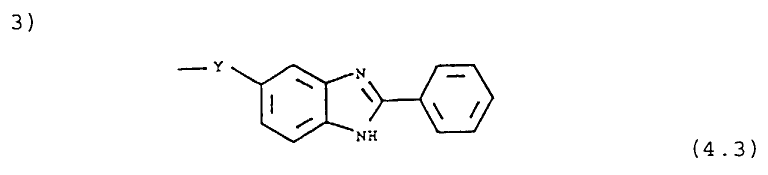

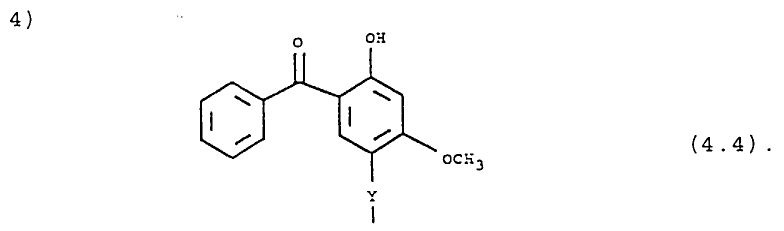

2. Neue Verbindungen nach Anspruch 1, die der Formel (1) oder der Formel (2) entsprechen, dadurch gekennzeichnet, daß die Gruppe R Alkylgruppen sind.

3. Neue Verbindungen nach Anspruch 2, dadurch gekennzeichnet, daß die Gruppen R Methyl, Ethyl, Propyl, n-Butyl, n-Octyl oder 2-Ethylhexyl bedeuten.

4. Neue Verbindungen nach Anspruch 3, dadurch gekennzeichnet, daß die Gruppen R Methylgruppen sind.

5. Neue Verbindungen nach einem der vorhergehenden Ansprüche, die der Formel (1) entsprechen, dadurch gekennzeichnet, daß die Gruppen B Alkylgruppen sind.

6. Neue Verbindungen nach Anspruch 5, dadurch gekennzeichnet, daß die Gruppen B die Gruppen Methyl, Ethyl, Propyl, n-Butyl, n-Octyl oder 2-Ethylhexyl bedeuten.

7. Neue Verbindungen nach Anspruch 6, dadurch gekennzeichnet, daß die Gruppen B Methylgruppen sind.

8. Neue Verbindungen nach einem der vorhergehenden Ansprüche, die der Formel (1) entsprechen, dadurch gekennzeichnet, daß r im Bereich von Null bis einschließlich 3 und s im Bereich von Null bis einschließlich 3 liegt.

9. Neue Verbindungen nach einem der Ansprüche 1 bis 4, die der Formel (2) entsprechen, dadurch gekennzeichnet, daß t+u im Bereich von 3 bis einschließlich 5 liegt.

10. Neue Verbindungen nach Anspruch 1, die der Formel (3) entsprechen, dadurch gekennzeichnet, daß die Gruppen R' Alkylgruppen sind, die unter den Gruppen Methyl, Ethyl, Propyl, n-Butyl, n-Octyl oder 2-Ethylhexyl ausgewählt sind.

11. Neue Verbindungen nach Anspruch 10, dadurch gekennzeichnet, daß die Gruppen R' Methylgruppen sind.

12. Neue Verbindungen nach einem der vorhergehenden Ansprüche, dadurch gekennzeichnet, daß in der zweiwertigen Gruppe -Y- p im Bereich von 1 bis einschließlich 3 liegt.

13. Neue Verbindungen nach einem der vorhergehenden Ansprüche, dadurch gekennzeichnet, daß in der zweiwertigen Gruppe -Y- R¹ Wasserstoff bedeutet.

14. Neue Verbindungen nach einem der vorhergehenden Ansprüche, dadurch gekennzeichnet, daß in der zweiwertigen Gruppe -Y- R₂ Wasserstoff oder Methyl bedeutet.

15. Neue Verbindungen nach einem der vorhergehenden Ansprüche, dadurch gekennzeichnet, daß A der Formel (4.1) entspricht.

16. Neue Verbindungen nach Anspruch 15, dadurch gekennzeichnet, daß X² Wasserstoff, Methyl, Methoxy oder -Y- bedeutet.

17. Neue Verbindungen nach einem der Ansprüche 15 oder 16, dadurch gekennzeichnet, daß X³ Wasserstoff oder -Y- bedeutet.

18. Neue Verbindungen nach einem der Ansprüche 1 bis 14, dadurch gekennzeichnet, daß A der Formel (4.2) entspricht.

19. Neue Verbindungen nach Anspruch 18, dadurch gekennzeichnet, daß X⁴ *tert*.-Butyl oder -Y- bedeutet.

20. Neue Verbindungen nach einem der Ansprüche 18 oder 19, dadurch gekennzeichnet, daß X⁵ Wasserstoff oder -Y- bedeutet.

21. Neue Verbindungen nach einem der Ansprüche 18 bis 20, dadurch gekennzeichnet, daß R³ Methyl oder *tert*.-Butyl bedeutet.

22. Neue Verbindungen nach einem der Ansprüche 1 bis 14, dadurch gekennzeichnet, daß A der Formel (4.3) entspricht.

23. Neue Verbindungen nach einem der Ansprüche 1 bis 14, dadurch gekennzeichnet, daß A der Formel (4.4) entspricht.

24. Verwendung der Verbindungen der Formeln (1) bis (3) nach einem der vorhergehenden Ansprüche als Sonnenschutzfilter, die im UV-A- und/oder UV-B-Bereich wirksam sind.

25. Kosmetische Zusammensetzung insbesondere zum Filter von UV-Strahlung, dadurch gekennzeichnet, daß sie in einem kosmetisch akzeptablen Träger eine wirksame Menge mindestens einer der Verbindungen nach einem der Ansprüche 1 bis 23 enthält.

26. Kosmetische Zusammensetzung nach Anspruch 25, dadurch gekennzeichnet, daß der kosmetisch akzeptable Träger mindestens eine Fettphase oder ein organisches Lösungsmittel enthält.

27. Kosmetische Zusammensetzung nach Anspruch 26, dadurch gekennzeichnet, daß der Träger in Form einer Emulsion vom Typ Öl-in-Wasser- oder Wasser-in-Öl-Emulsion und vorzugsweise als Öl-in-Wasser-Emulsion vorliegt.

28. Kosmetische Zusammensetzung nach einem der Ansprüche 25 bis 27, dadurch gekennzeichnet, daß der Gehalt an Filterverbindung und/oder Filterverbindungen im Bereich von 0,1 bis 20 Gew.-%, bezogen auf das Gesamtgewicht der Zusammensetzung, liegt.

29. Kosmetische Zusammensetzung nach Anspruch 28, dadurch gekennzeichnet, daß der Gehalt im Bereich von 0,5 bis 10 Gew.-% liegt.

30. Verfahren zum Schutz der Haut und/oder der Haare gegen UV-Strahlung und insbesondere gegen Sonnenlicht, dadurch gekennzeichnet, daß es darin besteht, auf die Haut und/oder die Haare eine wirksame Menge mindestens einer Verbindung oder mindestens einer Zusammensetzung nach einem der vorhergehenden Ansprüche aufzutragen.
